# NEUE EUROPÄISCHE PATENTSCHRIFT

(11) **EP 1 799 268 B2**
(45) Veröffentlichungstag und Bekanntmachung des Hinweises auf die Entscheidung über den Einspruch: **12.08.2015**
(45) Hinweis auf die Patenterteilung: 22.02.2012
(21) Anmeldenummer: 05806469.2
(22) Anmeldetag: 17.10.2005
(51) Int. Cl.: A61K 49/18, A61K 47/48, A61P 35/00, A61P 19/08, A61P 15/00, A61P 9/04, A61P 7/02, H01F 1/36, B82Y 5/00, A61P 25/00, B82Y 15/00

(54) **MAGNETISCHE PARTIKEL ZUR VERWENDUNG IN THERAPIE UND DIAGNOSTIK**
MAGNETIC PARTICLES FOR THERAPY AND DIAGNOSIS
PARTICULES MAGNETIQUES UTILISABLES EN THERAPIE ET POUR LE DIAGNOSTIC

(30) Priorität: 15.10.2004 DE 102004050583; 28.10.2004 DE 102004052533
(43) Veröffentlichungstag der Anmeldung: 27.06.2007
(73) Patentinhaber: Mykhaylyk, Olga, 80798 München (DE)
(72) Erfinder: Mykhaylyk, Olga, 80798 München (DE)
(74) Vertreter: Von Rohr Patentanwälte Partnerschaft mbB
(86) Internationale Anmeldenummer: PCT/EP2005/011124
(87) Internationale Veröffentlichungsnummer: WO 2006/042724

(56) Entgegenhaltungen:
- EP-A2- 1 800 774
- WO-A2-00/71169
- WO-A2-02/43708
- WO-A2-03/057359
- WO-A2-97/025073
- WO-A2-2004/081072
- DE-A1- 4 428 851
- DE-A1- 19 624 426
- US-A- 4 951 675
- US-A- 6 123 920
- US-A1- 2002 000 398
- US-A1- 2003 017 336
- US-B1- 6 541 039
- US-B2- 6 530 944
- MYKHAYLYK O M ET AL: "Signal transduction of erythrocytes after specific binding of ecdysterone and cholesterol immobilized on nanodispersed magnetite" JOURNAL OF MAGNETISM AND MAGNETIC MATERIALS, ELSEVIER SCIENCE PUBLISHERS, AMSTERDAM, NL, Bd. 225, Nr. 1-2, 2001, Seiten 226-234, XP004234950 ISSN: 0304-8853
- MYKHAYLYK O M ET AL: "USE OF ESR, MOESSBAUER SPECTROSCOPY, AND SQUID-MAGNETOMETRY FOR THE CHARACTERIZATION OF MAGNETIC NANOPARTICLES ON THE BASE OF METAL IRON AND ITS IMPLICATIONS IN VIVO" SCIENTIFIC AND CLINICAL APPLICATIONS OF MAGNETIC CARRIERS, XX, XX, 1997, Seiten 177-204, XP008062451
- GRAHAM C.D. ET AL.: "High-Field Magnetization of Metallic Glasses" IEEE TRANSACTIONS ON MAGNETICS, Bd. 29, Nr. 6, November 1993 (1993-11), Seiten 3457-3459, XP002414658
- MOSKOWITZ B.M.: 'Hitchiker's Guide to Magnetism', [Online] 29 September 2011, Gefunden im Internet: <URL:http://www.irm.umn.edu/hg2m/hg2m_a/hg2 m_a.html> [gefunden am 2011-09-29]
- WIKIPEDIA-AUSDRUCK ZUM STICHWORT "MAGNETIT"
- Bruce M Moskowitz, "Hitchhiker's Guide to Magnetism"
- A.F. HOLLEMAN ET AL.: 'Lehrbuch der Anorganischen Chemie', 1985, WALTER DE GRUYTER, BERLIN - NEW YORK Seiten 1136 - 1139
- B.M. MOSKOWITZ ET AL.: 'Global Earth Physics A Handbook of Physical Constants', 1995, AMERICAN GEOPHYSICAL UNION, WASHINGTON Seiten 346 - 350
- N. FELTIN ET AL.: 'New Technique for Synthesizing Iron Ferrite Magnetic Nanosized Particles' LANGMUIR Bd. 13, 1997, Seiten 3927 - 3933
- M.-P. PILENI: 'Magnetic Fluids: Fabrication, Magnetic Properties, and Organization of Nanocrystals' ADVANCED FUNCTIONAL MATERIALS Bd. 11, Nr. 5, Oktober 2001, Seiten 323 - 336
- A. K. GUPTA ET AL.: 'Synthesis and surface engineering of iron oxide nanoparticles for biomedical applications' BIOMATERIALS Bd. 26, 2005, Seiten 3995 - 4021
- P. A. DRESCO ET AL.: 'Preparation and Properties of Magnetite and Polymer Magnetite Nanoparticles' LANGMUIR Bd. 15, 1999, Seiten 1945 - 1951
- A. S. LÜBBE ET AL.: 'Clinical Applications of Magnetic Drug Targeting' JOURNAL OF SURGICAL RESEARCH Bd. 95, 2001, Seiten 200 - 206
- R. MASSART: 'Preparation of Aqueous Magnetic Liquids in Alkaline and Acidic Media' IEEE TRANSACTIONS ON MAGNETICS Bd. MAG-17, Nr. 2, 1981, Seiten 1247 - 1248
- D.K. KIM ET AL.: 'Synthesis and characterization of surfactant-coated superparamagnetic monodispersed iron oxide nanoparticles' JOURNAL OF MAGNETISM MATERIALS Bd. 225, 2001, Seiten 30 - 36
- Auszug aus: Cornell, Schwertmann, The Iron Oxides, Wiley; in Absatz ¬0023| des Streitpatents zitiert

## Beschreibung

Die vorliegende Erfindung betrifft Partikel gemäß dem Oberbegriff des Anspruchs 1, ein diese Partikel enthaltendes Arzneimittel bzw. Diagnostikum.

Wird einem Patienten ein Wirkstoff appliziert, erreicht dieser Wirkstoff üblicherweise nicht nur den gewünschten Wirkort, beispielsweise einen Tumor. Vielmehr verteilt sich der Wirkstoff nahezu im ganzen Körper. Um eine therapeutisch wirksame Konzentration am Zielort erreichen zu können, ist es deshalb aufgrund der Verteilung erforderlich, sehr hohe Dosen zu verabreichen. Dies führt jedoch zu unerwünschten Nebenwirkungen, die oftmals lebensbedrohliche Ausmaße annehmen können.

Um einen Wirkstoff gezielt an einem Wirkort anreichern zu können, ist es bekannt, den Wirkstoff mit magnetischen Trägem bzw. Partikeln als Arzneimittel bzw. Konjugat zu verbinden. Dem Patienten wird dann dieses Arzneimittel bzw. Konjugat verabreicht. Durch Anlegen eines externen, lokalen Magnetfeldes, das hauptsächlich auf den gewünschten Wirkort gerichtet ist, erfolgt dort eine gezielte Anreicherung. Der Wirkstoff kann dann gezielt dort seine Wirkung - also lokalisiert - entfalten, so daß insgesamt nur eine geringere Dosis des Wirkstoffs erforderlich ist. Dieses Verfahren wird unter anderem als "Magnetic Drug Targeting" bezeichnet.

Um ein effektives Magnetic Drug Targeting zu ermöglichen, müssen die magnetischen Partikel insbesondere optimale magnetische Eigenschaften aufweisen.

Aus der US 5,928,958 A, die den Ausgangspunkt der vorliegenden Erfindung bildet, sind superparamagnetische Partikel bekannt, bei denen superparamagnetische eisenhaltige Kerne eine Beschichtung bzw. Hülle aufweisen. Die Partikel sind für Drug-Targeting einsetzbar. Angaben zu den magnetischen Eigenschaften der Partikel fehlen.

DE4428851 offenbart eine Nanopartikel, dadurch gekennzeichnet, dass sie aus einem Eisen enthaltenden Kern, einem Primärcoat (Synthesepolymer) und einem Sekundärcoat (Targetpolymer) sowie gegebenenfalls pharmazeutischen Hilfsstoffen, Pharmaka und/oder Adsorptionsvermittlern besteht, zur Anwendung in der Therapie und Diagnostik. WO03057359 offenbart eine magnetische Partikel zur Anwendung in der Diagnostik, welche in einer Sauerstofffreien Atmosphäre hergestellt wird; bei einem Ausführungsbeispiel besteht der Kern aus Eisen und die sauerstoffdichte Hülle aus Graphit; bei einem anderen Ausführungsbeispiel besteht der Kern aus Magnetit (Fe₃O₄) und die sauerstoffdichte Hülle aus kondensierten Silikonen. Der Kern solcher Partikeln weist vorzugweise einen Durchmesser von 1 bis 50 nm auf.

Die US 4,951,675 A, die US 6,123,920 A und die US 2002/0000398 A1 offenbaren Partikel mit nanokristallinen magnetischen Kernen aus Eisenoxid, wie Fe₃O₄, γ-Fe₂O₃ oder Gemischen davon, die eine spezifische Sättigungsmagnetisierung der Partikel von meistens unter 100 Am²/kg Eisen, höchstens bis zu 107 Am²/kg Eisen, aufweisen. Die erreichte Sättigungsmagnetisierung ist im Vergleich zu der theoretisch erreichbaren Sättigungsmagnetisierung des nicht superparamagnetischen Bulk-Materials -bei Magnetit (Fe₃O₄) etwa 140 bis 145 Am²/kg Eisenrelativ gering. Dementsprechend sind die bisher erreichten magnetischen Eigenschaften von Nanopartikeln nicht optimal.

Weiter ist es für das magnetische Drug-Targeting wünschenswert, wenn die Partikel eine hohe Halbwertszeit im Blut eines Menschen aufweisen und biokompatibel sind.

Der vorliegenden Erfindung liegt daher die Aufgabe zugrunde, Partikel zur Anwendung in Diagnostik und Therapie, insbesondere für das Drug-Targeting, mit verbesserten magnetischen Eigenschaften sowie ein damit hergestelltes Arzneimittel bzw. Diagnostikum, ein Verfahren zur Herstellung dieser Partikel und eine Verwendung der Partikel zur Herstellung eines Arzneimittels oder Diagnostikums anzugeben, insbesondere wobei Nachteile des Standes der Technik vermieden werden können.

Die obige Aufgabe wird durch Partikel gemäß Anspruch 1, ein Arzneimittel bzw. Diagnostikum gemäß Anspruch 6 oder eine Verwendung gemäß Anspruch 7 gelöst. Vorteilhafte Weiterbildungen sind Gegenstand der jeweiligen Unteransprüche.

Die vorliegende Erfindung betrifft gemäß einem ersten Aspekt Partikel zur Anwendung in Diagnostik und/oder Therapie, insbesondere zur Bindung mit mindestens einem therapeutischen Wirkstoff, welche jeweils einen nanokristallinen magnetischen Kern und eine den Kern umschließende Hülle aufweisen, wobei der Kern zu mindestens 95 Gew.-% aus Eisenoxid besteht und die spezifische Sättigungsmagnetisierung der Partikel mindestens 110 Am²/kg Eisen beträgt. Der Begriff "magnetisch", wie er im Sinne der vorliegenden Erfindung verstanden wird, umfaßt sowohl magnetische Partikel als auch magnetisierbare Partikel. Die Partikel nach der vorliegenden Erfindung umfassen vorzugsweise ein Kernmaterial auf der Basis von Eisenoxidnanopartikeln mit einem mittleren Durchmesser von 3 bis 200 nm, vorzugsweise 10 bis 100 nm, insbesondere 20 bis 52 nm, und besitzen eine gegenüber dem Stand der Technik verbesserte spezifische Sättigungsmagnetisierung der Partikel von mindestens 110 Am²/kg, insbesondere mindestens 114 Am²/kg, vorzugsweise mindestens 117 Am²/kg, besonders bevorzugt mindestens 120 Am²/kg Eisen.

Die den nanokristallinen magnetischen Kern der erfindungsgemäßen Partikel umschließende Hülle besteht vorzugsweise aus einem biokompatiblen Material (z.B. Polymeren), vorzugsweise mit funktionellen Gruppen zur Anbindung, insbesondere kovalenten, ggf. lösbaren Anbindung, von therapeutischen Wirkstoffen (z.B. Antibiotikum, Antitumormittel etc.) bzw. Biomolekülen (Proteine, Polypeptide etc.), radioaktiven Substanzen, anorganischen Molekülen, Diagnostika o. dgl.

Das gewichtsbezogene Verhältnis Hülle/Eisen kann dabei in weiten Bereichen variieren, insbesondere von 0,05 bis 2,5, vorzugsweise 0,2 bis 1.

An die erfindungsgemäßen Partikel können therapeutische Wirkstoffe oder Biomoleküle angebunden sein, insbesondere über die äußere Hülle, und beispielsweise intravaskulär in Form einer Suspension zu diagnostischen oder therapeutischen Zwecken verabreicht werden; hierdurch wird ein verbessertes kinetisches Profil erreicht und kann ein effektives Targeting in bezug auf eine Magnetfeldanwendung - insbesondere bei hohen Gradienten - erreicht werden. Die erfindungsgemäßen Partikel können zu diagnostischen oder therapeutischen Zwecken verwendet werden, beispielsweise zur in-vivo-Diagnostik oder in-vivo-Therapie oder für in-vitro-Foschungszwecke.

Die erfindungsgemäßen Partikel werden in inerter Atmosphäre hergestellt. Hierdurch werden die magnetischen Eigenschaften des Kernmaterials durch unmittelbare Modifikation oder Stabilisierung der aktiven Oberfläche des Kerns auf Basis frisch hergestellter Eisenoxidnanokristalle durch eine passivierende oder im wesentlichen sauerstoffdichte Hüllschicht geschützt. Durch eine Nachbehandlung des Kernmaterials läßt sich die Beschichtung des Kerns erleichtern, beispielsweise durch Einführung funktioneller Gruppen (z.B. zur Verbesserung der Anbindung des Wirkstoffs bzw. Biomoleküls).

Die Verwendung der erfindungsgemäßen Partikel zur Anbindung von therapeutischen Wirkstoffen und Biomolekülen an der Oberfläche der Partikel liefert eine Reihe von Vorteilen, insbesondere in-vivo-Effekten, welche wie folgt zusammengefaßt werden können:
Die erfindungsgemäßen Partikel ermöglichen eine schnelle Adsorption oder auch Hemisorption von Wirkstoffen oder Biomolekülen (z. B. Doxorubicin oder Taxol) aus wäßrigen oder organischen Lösungen.
Die Beladungskapazität mit therapeutischen Wirkstoffen bzw. Biomoleküle, wie Doxorubicin, Taxol, Steroidhormonen etc., ist relativ hoch.
Aufgrund der Desorptionskinetik bzw. Hydrolyse sind die Konjugate aus Partikel und Wirkstoff bzw. Biomolekül besonders stabil.
Die Affinität in bezug auf spezielle Zelloberflächenrezeptoren (z. B. bei Steroiden, Doxorubicin etc.) bleibt erhalten. Ebenso die Möglichkeit in Zellen einzudringen.
Die Konzentration an therapeutischem Wirkstoff bzw. Biomolekül im gebundenen Zustand ist auf die jeweilige Anwendung optimiert, zum Beispiel im Hinblick auf den Hintergrund von Signaleffekten.
Wenn als therapeutischer Wirkstoff beispielsweise ein Antitumormittel bzw. ein Antikrebsmittel an die magnetischen Partikel angebunden ist, wird eine gute in-vitro-Antikrebsaktivität in verschiedenen Zellinien beobachtet. Die auf diese Weise mit therapeutischem Wirkstoff, insbesondere Antitumor- bzw. Antikrebsmittel, beladenen magnetischen Partikel zeigen in vivo keinerlei hämolytische Aktivität und stabilisieren überraschenderweise die Erythrozytenmembranen.

Das kinetische Profil der erfindungsgemäßen magnetischen Partikel mit dem darin gebundenen therapeutischen Wirkstoff bzw. Biomolekül ist - im Vergleich zu dem freien Wirkstoff bzw. Biomolekül oder im Vergleich zu polymeren WirkstoffBiomolekül-Konjugaten - im Hinblick auf die Verweildauer im Körper deutlich verlängert.

Die erfindungsgemäßen magnetischen Partikel ermöglichen ein effektives Targeting zu der Stelle eines angelegten Magnetfeldes mit hohem Gradienten. Die Mobilität der erfindungsgemäßen Nanopartikel in dem Magnetfeldgradienten ist derart, daß sich die Partikel über ein äußeres angelegtes Magnetfeld nach Gabe in den menschlichen Körper an die gewünschte Stelle hinleiten bzw. lokalisieren lassen. Hierdurch wird eine erhöhte Verfügbarkeit an der Stelle der Magnetfeldanwendung erreicht, so daß sich beispielsweise für den Fall, daß ein Antitumormittel an die erfindungsgemäßen Partikel gebunden ist, solche Partikel gezielt an die Stellen des Körpers bringen lassen, an denen der Tumor und/oder typischerweise Metastasen vorhanden ist bzw. sind (z. B. in Lunge, Leber und Milz). So kann auch gezielt verhindert werden, daß die Partikel in unerwünschte Bereiche (z. B. zu Herz oder Nieren) gelangen, so daß die Toxizität von Antitumormitteln in bezug auf Herz und Nieren sowie die Neurotoxizität deutlich reduziert werden können. Überraschenderweise sind die Effekte des Magnetfeldes in bezug auf die Pharmakokinetik nicht darauf beschränkt, diese an der Targetstelle zu lokalisieren.

Durch Erhöhung der Magnetfeldstärke und/oder des Gradienten an anderen Stellunge kann die Aufnahme des therapeutischen Wirkstoffs in stark durchbluteten Leber-, Milz- oder Lungengewebe vermindert bzw. verzögert werden. Dieser Effekt bietet neue Möglichkeiten, das Verteilungsprofil des an die magnetischen Partikel gebundenen Wirkstoffs gezielt zu modifizieren, und kann auch genutzt werden, um die Kinetik nichtmagnetischer Nanopartikel zu beeinflussen.

Die Herstellungsmöglichkeiten einheitlicher Metalloxidkolloide sind komplex und zahlreich. Bei Ausfällung aus homogenen Lösungen ist es wünschenswert, einen sogenannten Burst von Kernen (Nuclei) zu erhalten, die dann einheitlich wachsen können, um einheitlich gebildete Partikel mit enger Größenverteilung zu bilden. Komplexierungsreaktionen in Metallsalzlösungen werden durch geringfügige Änderungen in experimentellen Parametern stark beeinflußt, so zum Beispiel Temperatur, pH-Wert sowie Natur und Konzentration der Ionen. Die kolloidalen Partikel können also durch Phasentransformation des festen Ausgangspräzipitats in ihrer endgültigen Form erhalten werden. Einheitlich geformte Magnetitpartikel können beispielsweise durch Ausfällen von Eisen(II)hydroxid erhalten werden, welches dann in Gegenwart von Kaliumnitrat als mildem Oxidationsmittel zu Magnetit kristallisiert wird.

Unter Berücksichtigung der Tatsache, daß für eine Anwendung im menschlichen Körper die Trägersubstanz biokompatibel, insbesondere nichttoxisch, sein soll, kann magnetisches Kernmaterial auf Basis von magnetischem bzw. magnetisierbarem Eisenoxid verwendet werden.

Das magnetische Moment der Partikel ist kritisch im Hinblick auf das magnetische Drug-Targeting insofern, als die magnetische Kraft, die auf die magnetischen Partikel einwirkt, proportional zum magnetischen Moment ist. Bei niedrigem Magnetfeld ist die Kraft zunächst proportional zum Magnetfeld, zum Gradienten des Magnetfelds, zum Volumen der Partikel und zur magnetischen Suszeptibilität des Kernmaterials. Bei der Sättigungsmagnetisierung des Kernmaterials ist die Kraft proportional zu dem Modul des Magnetfeldgradienten, der Sättigungsmagnetisierung und dem Volumen der Partikel. Letzteres impliziert, daß die Sättigungsmagnetisierung des Kernmaterials das wichtigste Charakteristikum ist.

Magnetit (Fe₃O₄) hat als Bulk-Material eine spezifische Magnetisierung von bis zu 92 bis 100 mA²/kg Magnetit bei 300 K, d. h. 127 bis 138 mA²/kg Eisen (R.M. Cornell, U. Schwertmann, The Iron Oxides, Wiley, Weinhein, 1996).

Jedoch wird eine Abnahme der Sättigungsmagnetisierung der Nanopartikel mit abnehmendem Durchmesser beobachtet, verglichen zu den Eigenschaften des Bulk-Materials. Eine sogenannte magnetisch "tote" Oberflächenschicht des Kerns wird für die geringe Magnetisierung von Nanomaterialien verantwortlich gemacht. Sie nimmt mit abnehmenden Korndurchmesser überproportional relativ zum Kernvolumen zu und kann beispielsweise durch Oxidation, Hydration oder fehlerhafte magnetische Ordnung hervorgerufen werden. So ist die verhältnismäßige geringe Sättigungsmagnetisierung zu erklären, die bei dem eingangs genannten Stand der Technik bei Nanopartikeln aus Magnetit erreicht worden ist.

Die allgemeine Verfahrensweise des erfindungsgemäßen Verfahrens besteht darin, zunächst die nanokristallinen magnetischen Kerne, insbesondere durch Phasentransformation, herzustellen, die unter vorzugsweise inerter Atmosphäre anschließend unmittelbar mit einer passivierenden oder sauerstoffdichten Hülle versehen werden, welche die Oberfläche der Nanokristalle stabilisiert und modifiziert und die Anbindung von therapeutischen Wirkstoffen bzw. Biomolekülen ermöglicht.

Besondere Sorgfalt muß bei der Ausbildung bzw. beim Aufbringen der passivierenden oder sauerstoffundurchlässigen Hüllschicht auf die Nanokristalle angewendet werden; auch muß die Ausbildung und Vernetzung von Nanokristallaggregaten verhindert werden. Zu diesen Zwecken hat sich die Ultraschallbehandlung während der Modifizierungsbehandlung bzw. Ausbildung der Hüllschicht bestens bewährt.

Eine der Besonderheiten des erfindungsgemäßen Verfahrens liegt darin, daß die magnetischen Eigenschaften des Kernmaterials im wesentlichen bewahrt bleiben, insbesondere durch "Passivierung" der Oberfläche durch Modifizieren der aktiven Oberfläche frisch hergestellter Eisenoxidnanokristalle in einer inerten Atmosphäre unmittelbar nach Erhalt der Nanokristalle (Aufbringen einer Hülle). Überraschenderweise erlaubt es das erfindungsgemäße Verfahren, magnetische Partikel herzustellen, deren Sättigungsmagnetisierung dem Bullc-Material, insbesondere Bulk-Magnetit, so nah wie möglich kommt, insbesondere infolge der Passivierung der aktiven Stellen der Oberflächen der Nanokristalle, so daß auf diese Weise eine unerwünschte oder unkontrollierte Oxidation der Oberflächenschichten zumindest teilweise verhindert wird, was unerwünschterweise in Gegenwart von Sauerstoff und Feuchtigkeit geschieht und zur Bildung von überwiegend nichtmagnetischen oder antiferromagnetischen Oxiden und Hydroxiden an einer dünnen Oberflächenschicht führt. Die einzige im Anhang befindlichen Tabellen fassen die Eigenschaften der nach dem erfindungsgemäßen Verfahren hergestellten Teilchen entsprechend den Ausführungsbeispielen zusammen.

Die mittlere Kerngröße in der Tabelle wurde basierend auf TEM-Messungen, d.h. Messungen mit dem Transmissions-Elektronen-Mikroskop, unter der Annahme, daß die Partikelkerne Ellipsoid darstellen, bestimmt. Weiter wurde die mittlere Kristallitengröße in der Tabelle anhand von Röntgenuntersuchungen, basierend auf der Sherrer-Gleichung, bestimmt.

Die erfindungsgemäßen Partikel weisen monokristalline Kerne auf, die jeweils eine einzige magnetische Domäne bilden.

Typischerweise kann die Herstellung der erfindungsgemäßen Partikel wie folgt erfolgen: In einem ersten Schritt wird Eisen(II)hydroxid aus einer wäßrigen Eisen(II)sulfat-Lösung mit Kaliumhydroxid in Abwesenheit von Sauerstoff ausgefällt, anschließend wird das Eisen(II)hydroxid mit Kaliumnitrat einer milden Oxidation unterworfen, um eine Eisen(III)-Spezies einzuführen, gefolgt von einer Bildung von Magnetitkristallen bei einer Temperatur von mindestens 75 °C, insbesondere etwa 90 °C. Dann werden die Nanokristalle mit Wasser, aus dem zuvor Sauerstoff bzw. Luft durch Entgasen oder Durchblasen eines inerten Gases bzw. Stickstoff entfernt worden ist, gewaschen und anschließend - unmittelbar nach Erhalt der Nanokristalle - in einer zumindest im wesentlichen sauerstofffreien Atmosphäre an der Oberfläche modifiziert bzw. stabilisiert, insbesondere durch Aufbringen der Hülle. Die Hülle kann anschließend oder aber auch zu einem späteren Zeitpunkt noch mit funktionellen Gruppen versehen werden, insbesondere um ein Anbinden von therapeutischen Wirkstoffen oder Biomolekülen zu erleichtern.

Beispielsweise kann das erfindungsgemäße Verfahren gemäß einer typischen Ausfiihrungsform wie folgt durchgeführt werden: Vorzugsweise wird das gesamte Verfahren in einer zumindest im wesentlichen sauerstofffreien Atmosphäre durchgeführt, vorzugsweise unter einer Stickstoffatmosphäre. Zunächst wird Sauerstoff bzw. Luft aus dem Reaktionsreaktor und dem destillierten Wasser, welches zur Herstellung der nachfolgenden Lösungen verwendet wird, entfernt. Anschließend werden die erforderlichen Mengen an Eisen(II)sulfat, d. h. im allgemeinen FeSO₄ · 7H₂O, Natriumnitrat und Kaliumhydroxid getrennt voneinander in dem entgasten bzw. sauerstofffreien, destillierten Wasser gelöst. Dann wird eine Lösung des Stabilisierungsreagenzes (=Hüllschichtmaterials bzw. Modifizierungsmaterials) angesetzt, welches für die Beschichtung der Teilchen bzw. Ausstattung mit einer Hülle in eine sauerstofffreie Lösung überführt wird. Nachfolgend werden die Lösungen von Natriumhydroxid, Natriumnitrat, ein Teil Wasser und schließlich die Eisen(II)sulfat-Lösung bei Raumtemperatur schnell miteinander in der Reihenfolge, wie sie zuvor genannt worden sind, zusammengemischt, und die Reaktionsmischung wird ohne weiteres Rühren rasch aufgeheizt auf mindestens 75 °C, vorzugsweise auf etwa 90 °C, und für etwa 1,5 bis 4 Stunden, vorzugsweise 2 bis 4 Stunden, so belassen, damit die Nanokristalle wachsen können. Mit Hilfe eines Magnetabscheiders werden die erhaltenen Kristalle anschließend mit sauerstofffreiem Wasser gewaschen. Anschließend wird das überschüssige Waschwasser entfernt und die Eisenoxidkerne mit einer Lösung des Beschichtungsmaterials in Kontakt gebracht, um die Eisenoxidkerne mit einer modifizierenden bzw. stabilisierenden Hülle zu versehen, und die resultierende Reaktionsmischung wird anschließend etwa eine Stunde lang mit Ultraschall behandelt, dann mehrere Stunden bei periodischer Ultraschallbehandlung belassen. Anschließend wird das erhaltene Endprodukt gewaschen und in Suspension gelagert oder im Vakuum getrocknet und bis zu seiner Verwendung gelagert.

Die Modifizierungs- bzw. Stabilisierungsbehandlung kann beispielsweise auch eine Chemisorption von therapeutischen Wirkstoffen oder Biopolymeren mit geeigneten funktionellen Gruppen, die Kondensation von Organosilanen, die Pfropfpolymerisation von Monomeren, die durch die aktiven Stellen an der Oberfläche der frisch hergestellten magnetischen Nanokristalle initiiert werden kann, umfassen. Eine Vielzahl biokompatibler Polymere mit funktionellen Amino-, Hydroxil-, Thiol-, Phosphat-, Carboxylgruppen etc. kann zur Modifizierung und Stabilisierung der Oberfläche verwendet werden, hierunter auch Polysaccharide.

Polysaccharide und ihre Derivate werden oft für eine Reihe von Zwecken in der biologischen Anwendung verwendet, einschließlich der Beschichtung von magnetischen Nanomaterialien für biomedizinische Anwendungen.

Dextrane sind inhomogene Glukosepolymere (C₆H₁₀O₅)ₙ, die klinisch seit mehr als fünfzig Jahren für Plasmavolumenexpansion und als antithrombotische Substanzen verwendet werden. Die folgenden Eigenschaften der Dextrane machen sie für ihre Verwendung in diesem Bereich attraktiv:
1. Verlängerung der Langlebigkeit der Reagenzien bei Zirkulation bzw. im Blut;
2. Verlängerung der in-vitro-Stabilität und Abnahme der in-vivo-Immunogenizität von Proteinen und Enzymen;
3. hohe Löslichkeit in Wasser für die meisten kommerziell verfügbaren Dextrane mit geringem Grad bzw. Anteil an Verzweigung (0,5 %);
4. Stabilität unter leicht sauren und basischen Bedingungen;
5. große Anzahl an Hydroxylgruppen für die Konjugation bzw. Anbindung von Wirkstoffen bzw. Biomolekülen;
6. relativ geringe Kosten;
7. Historie der klinischen Verwendung.

Die Abwesenheit ionogener Gruppen ist sehr wichtig, um eine geringe Sorption von Wirkstoffen bzw. Biomolekülen auf der Carrier- bzw. Trägeroberfläche zu gewährleisten. Dextrane enthalten im allgemeinen Lactongruppen, welche im alkalischen Medium hydrolysieren, um Carboxylgruppen zu liefern, welche als Ankergruppen zur Pfropfung von Dextranpolymeren an der Oberfläche verwendet werden können. Der Rest der Carboxylgruppen kann leicht reduziert werden, z. B. durch Natriumborhydrid im alkalischen pH-Bereich.

Um die Agglomeration der Teilchen während der Umhüllung der Kerne zu verringern, wird die Konzentration an magnetischer Trägersuspension vorteilhafterweise unter 5 % gehalten.

Auf diese Weise kann das Polymer auf der Oberfläche der Nanokristalle bei alkalischem pH-Wert unter Verwendung der Reaktivität der Carboxylgruppen und der Donor-/Akpeptor-Wechselwirkung mit den Eisenatomen unter konstantem Ultraschalleintrag adsorbiert werden, wobei die Ultraschallbehandlung der Zerstörung von Aggregaten der Partikel dient. Anschließend kann die Reduktion überzähliger Carboxylgruppen unter Verwendung von Natriumborhydrid durchgeführt werden, gefolgt von einer Einführung von Epichlorhydrin zur Vernetzung des Dextrans in der adsorbierten Schicht, wobei diese Reaktion bis zu mehreren Stunden dauern kann und vorzugsweise unter konstantem Ultraschalleintrag erfolgt, um ein Vernetzen der Partikel zu minimieren. Der Gehalt an Vernetzung hängt stark von der Konzentration an Polymer und Vemetzungsreagenz ab.

Schema: Vernetzung von Dextran (D-OH) mit Epichlorhydrin

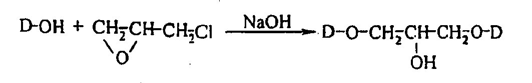

Nach der Herstellung der vorschlagsgemäßen Partikel kann zur Verwendung eine Oberflächenaktivierung, auch kurz Aktivierung genannt, erfolgen, wie beispielhaft nachfolgend beschrieben:

Die Oberflächenaktivierung des magnetischen Nanomaterials, welches mit einem biokompatiblen, Hydroxylgruppen oder Aminogruppen enthaltenden Biopolymer beschichtete Magnetitnanokristalle enthält, kann beispielsweise unter Verwendung einer Diisocyanatverbindung (z.B. 1,6-Hexaniethylendiisocyanat) und Tetrabutyltitanat als Katalysator zur Bildung von Urethanbindungen durchgeführt werden, insbesondere wie in der EP 0 052 365 A1 bzw. US 4,412,000 A beschieben, die hiermit ergänzend als Offenbarung eingeführt werden. Im Rahmen des Aktivierungsverfahrens sollten Vernetzungen der Nanopartikel vermieden werden. Zu diesem Zweck kann eine Ultraschallbehandlung während der Aktivierung durchgeführt werden.

Die Verwendung von Isocyanatverbindungen in Gegenwart eines ungiftigen Katalysator auf Basis von Titan, wie Tetrabutyltitanat (TBT), führt zur Bildung von Urethanbindungen, die hydrolysestabil sind. Weiterhin sind keine Cyanidionen vorhanden, und die Verwendung von hochsiedendem Isocyanat führt gleichermaßen zur Bildung eines Abstandhalters ("Spacers") zwischen dem Matrix- bzw. Vorlagepolymer und dem immobilisierten Molekül, was günstig in bezug auf die Aktivität ist und die Reaktivität der immobilisierten Biomoleküle gegenüber spezifischen Rezeptoren auf der Zelloberfläche gewährleistet.

Geeignete Lösemittel sind Aceton, Dichlormethan oder Frigen. Die Isocyanatverbindung kann ein Diisocyanat sein, beispielsweise Hexamethylendiisocyanat. Das biologisch aktive Material, welches an die aktivierten Träger (Partikel) gebunden werden soll, weist vorzugsweise nukleophile Gruppen auf, wie Amino-, Hydroxyl- und Thiolgruppen, welche mit einer Isocyanatgruppe reagieren können.

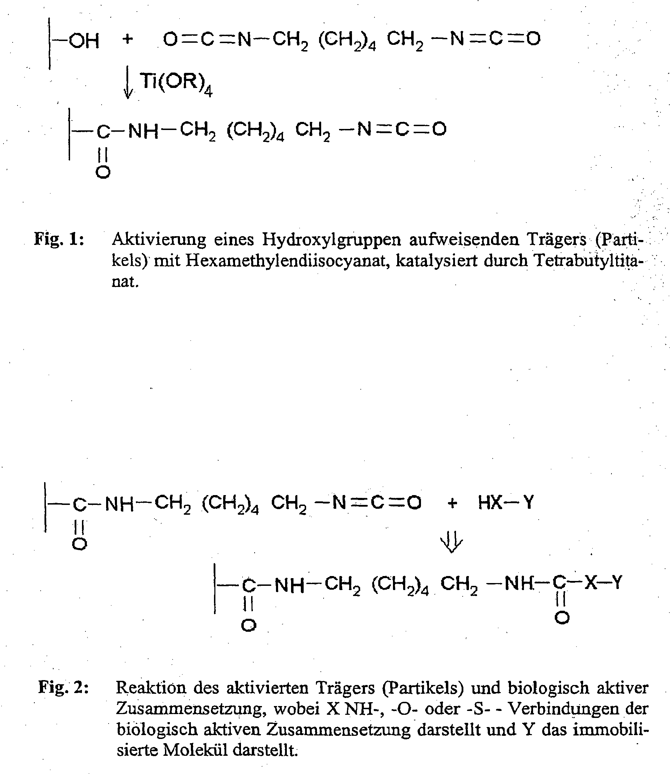

Die Aktivierungsreaktion erfolgt insbesondere gemäß Fig. 1.

Die Reaktion wird z.B. in einem wasserfreiem Medium durchgeführt. Wenn anschließend Wasser mit dem Isocyanatträger reagiert, werden die im Rahmen des Verfahrens nicht abgebauten ("weggespülten") Katalysatorreste sehr schnell hydrolysiert, und zwar schneller als die Hydrolyse der Isocyanatgruppen erfolgt. Im Vergleich zu dem Katalysator sind aliphatische Isocyanatgruppen nicht sehr hydrolyseempfindlich. Der aktivierte Träger ("Carrier") benötigt in bezug auf die Reaktion in wäßriger Lösung mit dem biologisch aktiven Material, welches nukleophile Gruppen, wie Amino-, Hydroxyl- und/oder Thiolgruppen enthält, keinen Katalysator.

Fig. 2 veranschaulicht die Reaktion des aktivierten Trägers (Partikels) mit der biologisch aktiven Verbindung.

Das Verfahren wurde in bezug auf die Aktivierung von magnetischem Nanomaterial, das auf Magnetit und/oder nanodispergierten Metallionen, wie Ferrite, basiert und Hydroxylgruppen enthaltende Beschichtungen aufweist, angepaßt und für die weitere Immobilisierung von erhältlichen Biomolekülen mit Hydroxyl- oder Aminogruppen verwendet.

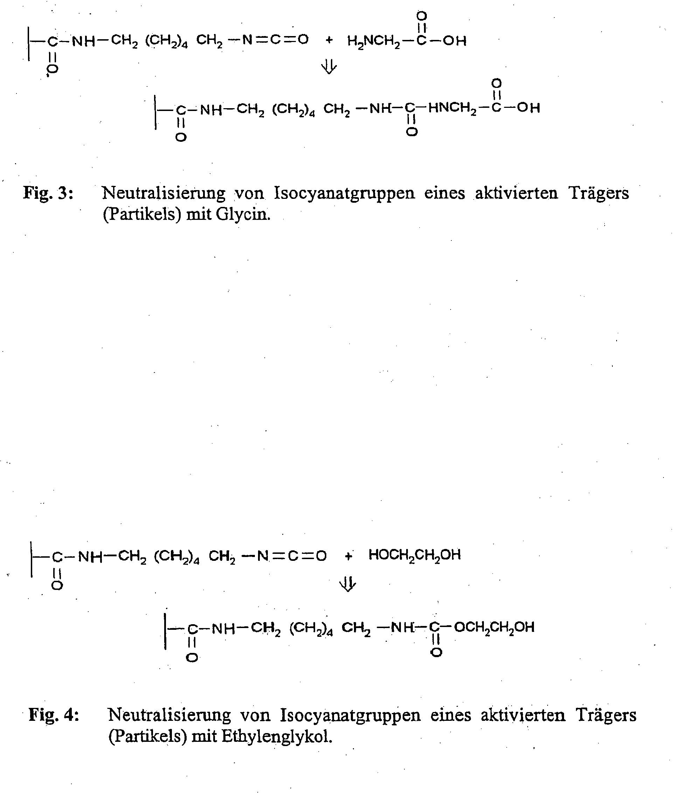

Kleine Moleküle, beispielsweise Glycin, können verwendet werden, um verbleibende aktive Gruppen, welche mit 1,6-Hexandiisocyanat nach der Immobilisierung der Biomoleküle aktiviert werden, auf der Oberfläche des Trägers zu neutralisieren. In diesem Fall reagieren Aminogruppen von Aminosäuren mit Isocyanatgruppen, wie in Fig. 3 gezeigt ist.

Bei diesem Vorgehen werden jedoch Carboxylgruppen auf der Oberfläche eingefügt, was zu einer Verringerung der Zirkulationszeit bzw. Verweildauer des Trägers (Partikels) im Blut führen kann. Aus diesem Grund sollte ein anderes kleines Molekül, wie Ethylenglykol, verwendet werden. Der Neutralisationsschritt kann gemäß Fig. 4 dargestellt werden.

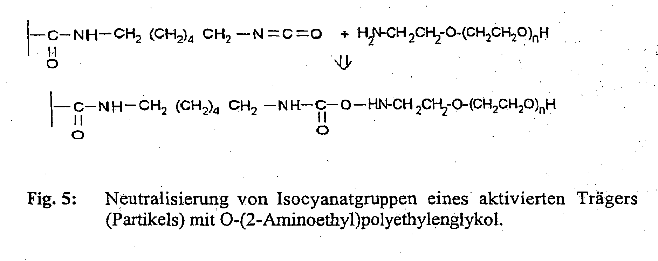

Um in einem Schritt sowohl die Neutralisierung von restlichen Isocyanatgruppen (zur Vermeidung einer weiteren Chemisorption von Molekülen mit nukleophilen Gruppen) zu erreichen, als auch die Oberfläche zu modifizieren, kann Polyethylenglykol verwenden werden. Aus dem Stand der Technik ist bekannt, daß Polyethylenglykol die Aufnahme durch retikuläre Endothelzellen verringern und die Halbwertzeit in bezug auf die Blutzirkulation verlängern kann. Das Reaktionsschema kann Fig. 5 entnommen werden.

Das Verfahren weist eine Reihe von Vorteilen auf: Durch das erfindungsgemäße Verfahren wird eine schnelle Chemisorption von Wirkstoffen aus wäßrigen oder organischen Lösungen gewährleistet.

Aufgrund der Desorptionskinetik sind die Konjugate aus Partikeln und Wirkstoffen bzw. Biomolekülen stabil.

Weiterhin wird durch das Verfahren gewährleistet, daß die Affinität des therapeutischen Wirkstoffes bzw. Biomoleküls in bezug auf spezielle Zelloberflächenrezeptoren erhalten bleibt.

Darüber hinaus ist die Konzentration an therapeutischem Wirkstoff bzw. Biomolekül im gebundenen Zustand in bezug auf die jeweilige Anwendung optimiert, beispielsweise im Hinblick auf den Hintergrund von Signaleffekten.

Wenn als therapeutischer Wirkstoff beispielsweise ein Antitumormittel bzw. ein Antikrebsmittel an die magnetischen Partikel gebunden ist, wurde eine gute in vivo-Antikrebsaktivität in zwei Zellinien beobachtet. Entsprechende Ergebnisse sind Tabelle 2 zu entnehmen, die Bezug auf Ausführungsbeispiele nimmt.

Schließlich weisen die mit Hilfe des Verfahrens mit einem therapeutischen Wirkstoff, insbesondere einem Antitumor- bzw. Antikrebsmittel, beladenen magnetischen Partikel in vivo keinerlei hämolytische Aktivität und stabilisieren überraschenderweise die Erythrozytenmembran.

### Ausführungsbeispiele:

Bei allen nachfolgenden Beispielen wurden die Kerne der erfindungsgemäßen Partikel unter Schutzgasatmosphäre, insbesondere Stickstoff, hergestellt und umhüllt.

Verwendete Abkürzungen:
HEMA= 2-Hydroxyethylmethacrylat oder Glykolmetacrylat
AA = Acrylamid
EGDMA= Ethylenglykoldimethacrylat
HES= Hydroxyethylstärke
γ-APS=γ-Aminopropyltriethoxysilan

### Beispiel 1a: Herstellung erfindungsgemäßer Partikel mit einer Hüllschicht durch spontane Adsorption von Dextran aus einer wäßrigen Lösung mit nachfolgender Vernetzung der Hüllschicht

### Herstellung der Lösungen und des Kernmaterials:

5 l destilliertes und entionisiertes Wasser wurde mit Stickstoff unter Stickstoffatmosphäre gespült, um den Sauerstoff zu entfernen.

Um eine 2,5 M KOH-Lösung zu erhalten, wurden 84,2 g KOH in 600 ml sauerstofffreiem Wasser gelöst. Die erhaltene Lösung wurde über einem Membranfilter mit 0,2 µm Porengröße filtriert.

Um eine 1 M FeSO₄-Lösung zu erhalten, wurden 60 g FeSO₄ · 7H₂O in 200 ml sauerstofffreiem Wasser gelöst und über einem Membranfilter mit 0,2 µm Porengröße gefiltert. Die Eisenkonzentration wurde bestimmt, und erforderlichenfalles wurde das Volumen angepaßt, um eine 1 M Eisenkonzentration in der Lösung zu erhalten.

Um eine 1 M KNO₃-Lösung zu erhalten, wurden 20,3 g KNO₃ in 200 ml sauerstofffreiem Wasser gelöst. Die Lösung wurde unter Verwendung eines Membranfilters mit 0,2 µm Porengröße filtriert.

Eine 1 M KOH-Lösung wurde hergestellt durch Zugabe von 375 ml Wasser zu 250 ml der 2,5 M KOH-Lösung.

0,25 M KOH-Lösung wurde hergestellt durch Zugabe von 150 ml 2,5 M KOH-Lösung zu 1350 ml Wasser.

10%ige Dextran-Lösung (Molgewicht 64.000 bis 76.000 der Firma Sigma-Aldrich) in 1 M KOH: 10 g Dextran wurden in 100 ml der 1 M KOH-Lösung unter Rühren gelöst. Die Lösung wurde unter Verwendung eines Membranfilters mit 0,8 µm Porengröße gefiltert.

1400 ml sauerstofffreies Wasser, 200 ml der 2,5 M KOH-Lösung und 200 ml der 1 M KNO₃-Lösung wurden in einem Reaktor unter Stickstoffatmosphäre gemischt. 225 ml der 1 M FeSO₄ · 7H₂O-Lösung wurden schnell zu der Mischung in den Reaktor hinzugegeben, und die Mischung wurde rasch auf 90 °C aufgeheizt und 2 Stunden lang bei 90 °C belassen. Dabei bildeten sich dann die Kerne in Form von Magnetit-Nanokristallen. Die Mischung wurde in ein 500-ml-Becherglas unter Verwendung einer peristaltischen Pumpe gefördert. Dann wurden die Magnetit-Nanokristalle magnetisch entfernt und sorgfältig dreimal mit 1 M KOH-Lösung unter Verwendung magnetischer Trenntechniken gewaschen. Das Volumen der Suspension mit den Magnetit-Nanokristallen wurde auf 100 ml mit 1 M KOH-Lösung angepaßt.

### Umhüllung (Modifikation) des Kernmaterials:

Unter Ultraschallbehandlung der resultierenden Magnetitsuspension (100 ml) wurden 100 ml der 10%igen Dextranlösung zu der 1 M KOH-Lösung hinzugegeben. Die Mischung wurde unter Ultraschallbehandlung 2 Stunden lang inkubiert. Dann wurden 100 mg NaBH₄ hinzugegeben, die Mischung auf 50 °C erhitzt, dann 1 Stunde lang unter Ultraschallbehandlung bei 50 °C belassen, gefolgt von einer Zugabe von 2,5 ml Epichlorhydrin und Inkubierung der Mischung bei 45 °C für eine Stunde. Das Produkt wurde sorgfältig mit 0,25 M KOH-Lösung unter Ultraschallbehandlung mit Wasser gewaschen und für die weitere Verwendung in Suspension gelassen. Falls erforderlich, kann das Produkt gefriergetrocknet werden. Die Ausbeute betrug 10,1 g an Partikeln.

### Beispiel 1b: Herstellung erfindungsgemäßer Partikel mit einer Hüllschicht modifiziert durch spontane Adsorption von Dextran aus einer wäßrigen Lösung mit nachfolgender Vernetzung der modifizierenden Hüllschicht

Gemäß einer anderen Ausführungsform des erfindungsgemäßen Verfahrens wird bei der Herstellung des Magnetitkernmaterials wie folgt vorgegangen: 42 g KOH wurden in 300 ml entgastem Wasser gelöst, 10,2 g KNO₃ wurden in 100 ml sauerstofffreiem Wasser gelöst; 15,9 g FeSO₄ · 7H₂O wurden in 100 ml sauerstofffreiem Wasser gelöst. Bei Raumtemperatur wurden 700 ml entgastes Wasser, 100 ml KOH-Lösung und 100 ml KNO₃-Lösung in einem Reaktor gemischt, dann 100 ml Eisen(II)sulfat-Lösung rasch hinzugegeben, und die Mischung wurde rasch auf 90 °C erhitzt, in einen Thermostaten überführt und 2 Stunden lang bei 90 °C gehalten, wobei sich Kerne aus Magnetit bildeten. Anschließend wurde das Magnetit abgetrennt und mit verdünnter KOH-Lösung (200 ml der hergestellten KOH-Lösung, verdünnt mit 1.800 ml sauerstofffreiem Wasser) unter Verwendung einer magnetischen Abscheidung gewaschen.

### Bildung der Hüllschicht

In 25 ml 1 M KOH-Lösung wurden 1,25 g Dextran (Molgewicht: 70 bis 76 kD) gelöst, gefolgt von einer Membranfiltration der Lösung. 25 ml der hergestellten Dextranlösung wurden zu dem gewaschenen Magnetit, das immer unter Stickstoffatmosphäre gehalten wurde, hinzugegeben und eine Stunde unter Ultraschallbehandlung belassen. Anschließend wurden 50 mg NaBH₄ und 25 ml einer 1 M KOH hinzugegeben. Dann wurde die Temperatur der Reaktionsmischung auf etwa 50 °C erhöht. Nach 30 Minuten wurden 0,5 ml Epichlorhydrin hinzugegeben. Die Reaktionsmischung wurde bei 50 °C und unter Ultraschallbehandlung 10 Stunden belassen. Das Produkt wurde sorgfältig mit 0,25 M KOH unter Ultraschallbehandlung und dann mit Wasser gewaschen und im Vakuum getrocknet. Die Ausbeute an erfindungsgemäßem Trägermaterial betrug 4,0 g Partikel.

### Beispiel 2: Herstellung erfindungsgemäßer Partikel mit einer Füllschicht mit vernetzter Hydroxyethylstärke

Die Herstellung des Kernmaterials wurde wie in Beispiel 1a oder 1b durchgeführt.

Die Umhüllung (Modifizierung des Kernmaterials) wurde wie in Beispiel 1a durchgeführt, jedoch mit dem Unterschied, daß Hydroxyethylstärke (der Firma Sigma-Aldrich) anstelle von Dextranpolymeren verwendet wurde. Die Produktausbeute betrug 9,8 g an trockenem Nanomaterial.

Bei einer zweiten Durchführung betrug die Ausbeute 10,0 g Partikel.

### Beispiel 3a: Herstellung erfindungsgemäßer Partikel mit einer Hüllschicht mit kondensiertem γ-Aminopropyltriethoxysilan

### Herstellung des Kernmaterials

1400 ml sauerstofffreies Wasser, 200 ml einer 1,5 M KOH-Lösung und 200 ml einer 2 M KNO₃-Lösung wurden in einem Reaktor unter Stickstoffatmosphäre miteinander vermischt. 200 ml 1 M FeSO₄ · 7H₂O-Lösung wurden rasch zu der Mischung in dem Reaktor hinzugegeben, und die Mischung wurde rasch auf 90 °C erhitzt und 2,5 Stunden bei dieser Temperatur belassen, wobei sich die Kerne bildeten. Das resultierende magnetische Nanomaterial wurde über eine peristaltische Pumpe in ein Becherglas gepumpt, die Magnetitnanokristalle wurden magnetisch abgetrennt und gründlich dreimal mit 0,05 M KOH-Lösung unter Verwendung magnetischer Abscheidemethoden bzw. Abtrennmethoden gewaschen. Ein Teil der konzentrierten Suspension (enthaltend etwa 700 mg des Trockenmaterials) wurden in ein Becherglas gegeben, mit Wasser gewaschen, im Vakuum getrocknet und zu Untersuchungszwecken auf magnetische Eigenschaften aufbewahrt. Das Volumen der restlichen Suspension wurde mit 0,05 M KOH-Lösung auf 100 ml angepaßt.

### Umhüllung (Modifizierung) des Kernmaterials:

100 ml Suspension mit der 0,05 M KOH-Lösung wurden ultraschallbehandelt, 100 ml einer 2%igen γ-Aminopropyltriethoxysilan-Lösung in 0,05 M KOH wurden hinzugegeben, wobei eine Ultraschallbehandlung 10 Minuten lang durchgeführt wurde.

Das Becherglas wurde in einen Thermostaten eingebracht, und die Temperatur wurde auf 90 °C eingeregelt. Nach einer halben Stunde bei 90 °C wurde die Mischung über Nacht in dem ausgeschalteten Thermostaten belassen. Am nächsten Morgen wurde das Produkt gründlich mit sterilem Wasser unter milder Ultraschallbehandlung und dann mit Aceton gewaschen und in Acetonsuspension aufbewahrt. Die Ausbeute betrug 5,3 g Partikel.

### Beispiels 3b: Herstellung erfindungsgemäßer Partikel mit einer Hüllschicht mit kondensiertem γ-Aminopropyltriethoxysilan

### Herstellung des Kernmaterials

1450 ml sauerstofffreies Wasser, 200 ml einer 1,25 M KOH-Lösung und 200 ml einer 2 M KNO₃-Lösung wurden in einem geschlossenen Reaktor unter Stickstoffatmosphäre miteinander gemischt. 150 ml einer 1 M FeSO₄ · 7H₂O-Lösung wurden rasch zu der Mischung in dem Reaktor hinzugegeben. Die Mischung wurde rasch auf 90 °C erhitzt und 2,5 Stunden lang bei dieser Temperatur zur Bildung der Kerne gehalten. Das resultierende magnetische Nanomaterial wurde über eine peristaltische Pumpe in ein Becherglas gepumpt, die Magnetitnanokristalle wurden magnetisch abgetrennt und gründlich dreimal mit 0,05 M KOH-Lösung unter Verwendung magnetischer Abtrennmethoden gewaschen. Ein Teil der konzentrierten Suspension (enthaltend etwa 300 mg Trockenmaterial) wurden in ein Becherglas überführt, mit Wasser und Aceton gewaschen, im Vakuum getrocknet und zu Untersuchungszwecken auf magnetische Eigenschaften aufbewahrt. Das Volumen der restlichen Suspension wurde auf 100 ml mit 0,05 M KOH-Lösung angepaßt.

### Umhüllung (Modifizierung) des Kernmaterials:

100 ml der vorgenannten Suspension mit der 0,05 M KOH-Lösung wurden ultraschallbehandelt. 100 ml 2%iger γ-Aminopropyltriethoxysilan-Lösung in 0,05 M KOH wurde hinzugegeben und 10 Minuten lang einer Ultraschallbehandlung ausgesetzt. Das Becherglas wurde in einen Thermostaten eingebracht, und die Temperatur wurde auf 90 °C eingeregelt. Nach einer halben Stunde bei 90 °C wurde die Mischung über Nacht in dem ausgeschalteten Thermostaten belassen, und am nächsten Morgen wurde das Produkt gründlich mit sterilem Wasser unter Ultraschallbehandlung und dann mit Aceton gewaschen und in der Acetonsuspension gelagert. Die Ausbeute an Partikeln betrug 7,0 g des trockenen Nanomaterials.

### Beispiel 3c: Herstellung erfindungsgemäßer Partikel mit einer Hüllschicht mit kondensiertem γ-Aminopropyltriethoxysilan

Die Herstellung des Kernmaterials wurde wie in Beispiel 3a durchgeführt.

Die Umhüllung (Modifizierung) des Kernmaterials wurde wie in Beispiel 3a durchgeführt.

### Beispiel 3d: Herstellung erfindungsgemäßer Partikel mit einer Hüllschicht mit kondensiertem γ-Aminopropyltriethoxysilan.

Die Herstellung des Kernmaterials wurde wie Beispiel 1b durchgeführt.

Zur Umhüllung (Modifizierung) des Kernmaterials wurden 2,5 m γ-Aminopropyltriethoxysilan in 50 ml sauerstofffreiem Wasser hinzugegeben und in einem Thermostat bei 90 °C unter Ultraschallbehandlung 1 Stunde lang wärmebehandelt und für 12 Stunden in dem Thermostaten belassen. Dann wurde das Produkt mit Wasser unter Ultraschallbehandlung gewaschen und im Vakuum getrocknet. Die Ausbeute an Partikeln betrug 4,5 g.

### Beispiel 4: Herstellung erfindungsgemäßer Partikel mit einer Hüllschicht mit kondensiertem γ-Aminopropyltriethoxysilan mit nachfolgender Chemisorption von oxidiertem HES

Die Herstellung des Kernmaterials wurde wie in Beispiel 3a durchgeführt.

### Umhüllung (Modifizierung) des Kernmaterials:

Eine erste Umhüllung (Modifikation) der Oberfläche des frisch hergestellten Magnetitnanomaterials wurde wie in Beispiel 3a durchgeführt.

Eine Lösung von modifiziertem HES wurde durch Lösen von 10 g HES in 100 ml sauerstoff freiem Wasser hergestellt, anschließend wurde die Lösung über einen 0,2 µm Membranfilter gefiltert. 14 ml 0,4 M NaIO₄-Lösung in sauerstoff freiem Wasser wurden hinzugegeben, und die Mischung wurde 2 Stunden lang bei Raumtemperatur inkubiert. Anschließend wurden 100 ml Suspension der Magnetitnanokristalle in 0,05 M KOH-Lösung mit der Lösung des modifizierten HES gemischt, die Suspension wurde ultraschallbehandelt und 2 Stunden lang bei 50 °C unter Ultraschallbehandlung belassen. Das Produkt wurde sorgfältig mit sterilem Wasser unter milder Ultraschallbehandlung und dann mit Aceton gewaschen. Die Ausbeute an Partikeln betrug 7,5 g des trockenen Nanomaterials.

### Beispiel 5: Herstellung erfindungsgemäßer Partikel mit einer Hüllschicht mit Chitosan durch Chemisorption

Die Herstellung des Kernmaterials wurde wie in Beispiel 1b durchgeführt.

Zur Umhüllung (Modifizierung) des Kernmaterials wurden 50 ml 5%iger Chitosanlösung in 10 % einer sauerstofffreien Essigsäurelösung zu der frisch hergestellten Magnetitsuspension hinzugegeben und 10 Stunden lang unter Ultraschallbehandlung belassen. Anschließend wurde das Produkt mit 10%iger Essigsäure gewaschen, gefolgt von einem Waschen mit Wasser und eine Trocknung unter Vakuum. Die Ausbeute an Partikeln betrug 4,2 g des Trockenmaterials.

### Beispiels 6: Herstellung erfindungsgemäßer Partikel mit einer Hüllschicht durch Pfropfpolymerisation von Monomeren

### Herstellung des Kernmaterials:

Bei Raumtemperatur wurden 690 ml sauerstofffreies Wasser, 110 ml 1 M KOH-Lösung und 100 ml 2 M KNO₃-Lösung in einem Reaktionsgefäß gemischt. Dann wurden rasch 100 ml 0,5 M Eisen(II)sulfat-Lösung hinzugegeben, und die Mischung wurde rasch auf 90 °C erhitzt, in einen Thermostaten überführt und 2 Stunden lang bei 90 °C wärmebehandelt, wobei Kerne in Form von Magnetit gebildet wurden. Anschließend wurde das Magnetit abgetrennt und mit sauerstoff freien Wasser und Dimethylformamid unter Verwendung magnetischer Abtrenntechniken gewaschen.

### Umhüllung (Modifizierung) des Kernmaterials:

Unter einer Stickstoffatmosphäre wurde eine Lösung aus 1,2 g Acrylamid, 1,2 g 2-Hydroxyethylmethacrylat und 0,1 g Ethylenglykoldimethacrylat in 50 ml Dimethylformamid zu 50 ml der frisch hergestellten Magnetitsuspension in N,N-Dimethylformamid hinzugegeben und 2 Stunden lang bei 35 °C einer Ultraschallbehandlung unterzogen. Anschließend wurde das Produkt mit Dimethylformamid gewaschen. Die Ausbeute an Partikeln betrug 3,5 g des Trockenmaterials.

Die weiteren Beispiele 7 bis 18 beschreiben die Herstellung von bioaktiven Substanzen aus den erfindungsgemäßen Partikeln bzw. deren Verwendung:

### Beispiel 7: Partikel mit immobilisiertem DOX

Die Herstellung des Kernmaterials und der ersten (primären) Umhüllung (Modifizierung) des Kernmaterials erfolgte wie in Beispiel 1a.

Aktivierung des magnetischen Nanomaterials:

### Herstellung der Lösungen:

Zur Herstellung von trockenem bzw. wasserfreiem Dichlormethan wurden 300 ml Dichlormethan zunächst mit konzentrierter Schwefelsäure, dann mit Wasser, anschließend mit einer Natriumcarbonatlösung und dann wieder mit Wasser gewaschen und nachfolgend mit gebranntem Calciumchlorid getrocknet und über Phosphorpentoxid destilliert. Das Lösemittel wurde bis zur Verwendung in einem dunklen Kolben unter Trockenatmosphäre aufbewahrt.

Zur Herstellung einer 2%igen Tetrabutyltitanat (TBT)-Lösung in Dichlormethan wurden 1 g TBT (244112, von Sigma-Aldrich) in trockenem Dichlormethan gelöst und das Volumen wurde mit trockenem Dichlormethan auf 50 ml eingestellt.

### Aktivierungsverfahren:

Zur Oberflächenaktivierung wurden 5 g des magnetischen Nanomaterials, welches wie in Beispiel 1a mit einem vernetzten Dextran umhüllt (modifiziert) wurde, in 50 ml trockenes Dichlormethan gegeben, gefolgt von einer 10minütigen Ultraschallbehandlung. Anschließend wurden 50 ml der 2%igen TBT-Lösung in Dichlormethan zugegeben, und die Dispension wurde für 10 Minuten unter Ultraschallbehandlung inkubiert.

Anschließend wurden 3 ml 1,6-Hexandiisocyanat (D2026, von Sigma) tröpfchenweise hinzugegeben, und die Mischung wurde 2 Stunden lang unter milder Ultraschallbehandlung inkubiert. Anschließend wurde das Produkt mit trockenem Dichlormethan gewaschen und unter Verwendung einer Gefriertrocknungsvorrichtung (ein Freeze-Diyer-System) getrocknet. Die Ausbeute an aktiviertem magnetischem Nanomaterial betrug 4,92 g.

### DOX-Chemisorption:

Zur Herstellung einer wäßrigen Doxorubicinhydrochlorid (DOX)-Lösung wurden 10 mg DOX (D1515, von Sigma-Aldrich) in 10 ml doppelt destilliertem und entionisiertem Wasser gelöst. Die resultierende Lösung wies einen pH-Wert von 6,02 auf.

9,5 ml der wäßrigen DOX-Lösung wurden zu 0,2 g des aktivierten magnetischen Nanomaterials gegeben. Die Suspension wurde bei Raumtemperatur unter Ultraschallbehandlung inkubiert. Zur Verfolgung der DOX-Chemisorption an der Oberfläche des aktivierten Nanomaterials wurden Proben des Überstandes entnommen. Nach 2 Stunden wurde das Produkt mit Hilfe von Magnetismus bzw. magnetisch abgetrennt und sorgfältig mit Wasser gewaschen.

### Neutralisierung der restlichen bzw. verbliebenen Isocyanatgruppen nach DOX-Chemisorption:

Zur Neutralisierung der restlichen Isocyanatgruppen nach DOX-Chemisorption wurden 50 ml Ethylenglykol in 5 ml doppelt destilliertem und entionisiertem Wasser gelöst und 2 Stunden lang mit dem gewaschenen magnetischen Nanomaterial unter milder Ultraschallbehandlung inkubiert. Anschließend wurde das Material erneut mit Wasser und mit Aceton gewaschen, magnetisch abgetrennt und im Vakuum getrocknet.

Die Konzentration an immobilisiertem DOX betrug in dem Produkt 6,62 mg DOX/g Träger (Carrier).

### Beispiel 8: Partikel mit immobilisiertem DOX

Die Herstellung des Kernmaterials und der primären Umhüllung (Modifizierung) der Oberfläche erfolgte wie in Beispiel 2a.

### Aktivierung des magnetischen Nanomaterials:

5 g des wie in Beispiel 2 hergestellten Nanomaterials wurden wie in Beispiel 7 aktiviert.

### DOX-Chemisorption:

Zur Herstellung der wäßrigen DOX-Lösung wurden 8,2 mg DOX (D1515, von Sigma-Aldrich) in 8,9 ml doppelt destilliertem und entionisiertem Wasser gelöst. Die resultierende Lösung wies einen pH-Wert von 6,015 auf.

8,9 ml der hergestellten DOX-Lösung wurden zu 0,2 g aktiviertem magnetischem Nanomaterial gegeben. Die Suspension wurde bei Raumtemperatur unter Ultraschallbehandlung inkubiert. Zur Verfolgung der DOX-Chemisorption an der Oberfläche des aktivierten Nanomaterials wurden Proben des Überstandes entnommen. Nach zwei Stunden wurde das Produkt magnetisch abgetrennt und sorgfältig mit Wasser gewaschen.

### Neutralisierung der restlichen Isocyanatgruppen nach DOX-Chemisorption:

Zur Neutralisierung der restlichen Isocyanatgruppen nach DOX-Chemisorption wurden 50 ml Ethylenglykol in 5 ml doppelt destilliertem und entionisiertem Wasser gelöst und 2 Stunden lang mit gewaschenem magnetischem Nanomaterial unter Ultraschallbehandlung inkubiert. Anschließend wurde das Material erneut mit Wasser und Aceton gewaschen, magnetisch abgetrennt und unter Vakuum getrocknet.

Die Konzentration an immobilisiertem DOX betrug in dem Produkt 8,64 mg DOX/g Träger (Carrier).

### Beispiel 9: Partikel mit immobilisiertem DOX

Die Herstellung des Kernmaterials und der primären Umhüllung (Modifikation) der Oberfläche erfolgte wie in Beispiel 2.

### Aktivierungsverfahren:

Zur Aktivierung der Oberfläche wurden 5 g magnetisches Nanomaterial, welches mit vernetztem HES wie in Beispiel 2b modifiziert wurde, zu 50 ml trockenem Dichlormethan gegeben, gefolgt von einer 10miliütigen Ultraschallbehandlung. 50 ml einer 2%igen TBT-Lösung wurden zugegeben, und die Suspension wurde 10 Minuten lang inkubiert.

Anschließend wurden 20 ml einer Lösung von 0,5 g PEG-IC in Dichlormethan und 2 ml 1,6-Hexandüsocyanat (D2026, von Sigma) tröpfchenweise hinzugegeben und die Mischung wurde 3 Stunden lang unter periodischer und milder Ultraschallbehandlung inkubiert. Anschließend wurde das Produkt mit trockenem Dichlormethan gewaschen und unter Verwendung einer Gerfriertrocknungsvorrichtung getrocknet.

### DOX-Chemisorption:

Zur Herstellung einer wäßrigen DOX-Lösung wurden 19,4 mg DOX (D1515, von Sigma-Aldrich) in 20 ml doppelt destilliertem und entionisiertem Wasser gelöst. Die resultierende Lösung wies einen pH-Wert von 6,5 auf.

20 ml der so hergestellten DOX-Lösung wurden zu 0,40 g des aktivierten magnetischen Nanomaterials gegeben. Die Suspension wurde bei Raumtemperatur unter Ultraschallbehandlung inkubiert. Zur Verfolgung der DOX-Chemisorption an der Oberfläche des aktivierten Nanomaterials wurden Proben des Überstandes entnommen. Nach 3 Stunden wurde das Produkt magnetisch abgetrennt und sorgfältig mit Wasser gewaschen.

### Neutralisierung der restlichen Isocyanatgruppen nach DOX-Chemisorption:

Zur Neutralisierung der restlichen Isocyanatgruppen nach DOX-Chemisorption wurden 100 ml Ethylenglykol in 10 ml doppelt destilliertem und entionisiertem Wasser gelöst und 2 Stunden lang mit dem gewaschenen magnetischen Nanomaterial unter milder Ultraschallbehandlung inkubiert. Anschließend wurde das Material erneut mit Wasser und Aceton gewaschen, magnetisch abgetrennt und im Vakuum getrocknet.

Die Konzentration an immobilisiertem DOX betrug in dem Produkt 27 mg DOX/g Träger (Carrier).

### Beispiele 10 bis 14: Partikel mit verschiedenen Konzentrationen des immobilisierten DOX

Die Herstellung des Kernmaterials und der primären Umhüllung (Modifizierung) der Oberfläche erfolgte wie in Beispiel 3a.

### Aktivierung des magnetischen Nanomaterials:

### Herstellung der Reagenzien:

Zur Herstellung einer 1%igen TBT-Lösung in trockenem Dichlormethan wurden 1 g TBT in trockenem Dichlormethan gelöst und das Volumen wurde mit trokkenem Dichlormethan auf 100 ml eingestellt.

Zur Herstellung einer 1%igen HES-Lösung in sterilem, destilliertem und entionisiertem Wasser wurden 0,5 HES in 50 ml sterilem, destilliertem und entionisiertem Wasser gelöst. Die erhaltene Lösung wurde über einem Membranfilter mit 0,2 µm Porengröße filtriert.

5 g des gemäß Beispiel 3a mit γ-Aminopropyltriethoxysilan modifizierten magnetischen Nanomaterials wurden zu 50 ml trockenem Dichlormethan gegeben, gefolgt von einer 10minütigen Ultraschallbehandlung. 50 ml der 1%igen TBT-Lösung wurden zugegeben, und die Suspension wurde 10 Minuten lang inkubiert.

Anschließend wurden 0,5 ml 1,6-Hexandiisocyanat (D2026, von Sigma) tröpfchenweise hinzugegeben und die Mischung wurde 6 Stunden lang unter periodischer und milder Ultraschallbehandlung inkubiert. Dann wurde das Produkt mit trockenem Dichlormethan gewaschen und unter Verwendung einer Gerfriertrocknungsvorrichtung getrocknet.

4,5 g des Produktes wurden zu 50 ml sterilem Wasser gegeben, gefolgt von einer Ultraschallbehandlung. Anschließend wurden 50 ml der 1%igen HES-Lösung zugegeben, und die Mischung wurde 6 Stunden lang bei Raumtemperatur unter milder Ultraschallbehandlung inkubiert. Das Produkt wurde magnetisch abgetrennt und sorgfältig mit Wasser und anschließend mit Aceton gewaschen und im Vakuum getrocknet.

4,0 g des resultierenden Materials wurden in 50 ml trockenem Dichlormethan ultraschallbehandelt und 50 ml der 1%igen TBT-Lösung wurden zugegeben. Die Suspension wurde 10 Minuten lang unter milder Ultraschallbehandlung inkubiert. Anschließend wurden 0,5 g 1,6-Hexandiisocyanat tröpfchenweise hinzugegeben, und die Mischung wurde 6 Stunden lang bei Raumtemperatur unter milder Ultraschallbehandlung inkubiert. Das Produkt wurde magnetisch abgetrennt, mit trockenem Dichlormethan gewaschen und getrocknet.

### DOX-Chemisorption:

### Wäßrige DOX-Lösungen:

Zur Herstellung der DOX-Lösung gemäß Beispiel 10 wurden 12,2 mg DOX in 15 ml doppelt destilliertem und entionisiertem Wasser gelöst. Die resultierende Lösung wies einen pH-Wert von 5,929 auf.

Zur Herstellung der DOX-Lösung gemäß Beispiel 11 wurden 3,3 ml der DOX-Lösung gemäß Beispiel 10 in 14 ml doppelt destilliertem und entionisiertem Wasser gelöst.

Zur Herstellung der DOX-Lösung gemäß Beispiel 12 wurden 6 ml der DOX-Lösung gemäß Beispiel 11 in 26 ml doppelt destilliertem und entionisiertem Wasser gelöst.

Zur Herstellung der DOX-Lösung gemäß Beispiel 13 wurden 0,2 ml der DOX-Lösung gemäß Beispiel 10 mit doppelt destilliertem und entionisiertem Wasser auf 10 ml eingestellt.

Zur Herstellung der DOX-Lösung gemäß Beispiel 14 wurden 0,05 ml DOX-Lösung gemäß Beispiel 10 mit doppelt destilliertem und entionisiertem Wasser auf 10 ml eingestellt.

Einzelheiten sind Tabelle 3 zu entnehmen.

Zur Durchführung der DOX-Chemisorption auf der Oberfläche der aktivierten Probe 3a, welche in Tabelle 3 in bezug auf den aktivierten Träger (Carrier) angegeben ist, wurde die angegebene Menge der jeweiligen DOX-Lösung zugegeben. Die Suspension wurde bei Raumtemperatur unter Ultraschallbehandlung mit der Inkubationszeit T_{inc} inkubiert. Zur Verfolgung der DOX-Chemisorption an der Oberfläche des aktivierten Nanomaterials wurden Proben des Überstandes entnommen. Nach 3 Stunden wurde das Produkt magnetisch abgetrennt und sorgfältig mit Wasser gewaschen.

### Neutralisierung der restlichen Isocyanatgruppen auf der Oberfläche der Referenzproben und nach DOX-Chemisorption:

450 ml O-(2-Aminoethyl)polyethylenglykol 3.000 (von Sigma) wurden in 16 ml sterilem, doppelt destillierten und entionisiertem Wasser gelöst, um eine 2,5%ige Lösung zu erhalten.

Zu 0,25 g der aktivierten Probe 3a als Referenzprobe oder zu dem gewaschenen feuchten Material nach DOX-Chemisorption werden 10 ml Wasser und 2 ml der wäßrigen, 2,5%igen O-(2-Aminoethyl)polyethylenglykol-Lösung zugegeben. Nach einer Inkubation von 2 Stunden bei Raumtemperatur wurden die Produkte sorgfältig mit Wasser und Aceton gewaschen und getrocknet.

Die Konzentration an immobilisiertem DOX in den Produkten variierte von 0,16 mg bis 25,1 mg DOX/g Träger (Carrier), wie es der Tabelle 3 zu entnehmen ist.

### Beispiel 15: Partikel mit immobilisierten DOX

Die Herstellung des Kernmaterials und der primären Umhüllung (Modifizierung) der Oberfläche erfolgte wie in Beispiel 4.

### DOX-Chemisorption:

Zur Herstellung von wäßrigen DOX-Lösungen wurden 18,4 mg DOX in 18 ml doppelt destilliertem und entionisiertem Wasser gelöst. Die resultierende Lösung wies einen pH-Wert von 6,475 auf.

Zur Herstellung von 2,5 % (w/v) O-(2-Aminoethyl)polyethylenglykol 3.000 in Krebspuffer wurden 0,3 g O-(2-Aminoethyl)polyethylenglykol 3.000 in 12 ml Krebspuffer bei einem pH-Wert von 5,4 gelöst.

Zur Herstellung von 1 % (w/v) Glycin in Wasser wurden 0,12 g Glycin in 12 ml sterilem, doppelt destilliertem und entionisiertem Wasser gelöst.

12 ml der hergestellten DOX-Lösung wurden zu 0,253 g des aktivierten magnetischen Nanomaterials zugegeben. Die Suspension wurde bei Raumtemperatur unter Ultraschallbehandlung inkubiert. Um die DOX-Chemisorption an der Oberfläche des aktivierten Nanomaterials zu verfolgen, wurden Proben des Überstandes entnommen. Nach einer 1 stündigen Inkubation wurden 2 ml der 2,5%igen (w/v) O-(2-Aminoethyl)polyethylenglykol-Lösung 3.000 in Krebspuffer zugegeben, gefolgt von einer erneuten Inkubation unter milder Ultraschallbehandlung der Suspension für eine weitere Stunde. Anschließend wurden 2 ml der 1%igen Glycinlösung zugegeben, gefolgt von einer 1 stündigen Inkubation. Nach der gesamten, 3-stündigen Inkubation wurde das Produkt magnetisch abgetrennt und sorgfältig mit Wasser gewaschen und gefriergetrocknet.

Die Konzentration an immobilisiertem DOX betrug in dem Produkt 1,8 mg DOX/g Träger (Carrier).

### Beispiel 16: Partikel mit immobilisiertem DOX

Die Herstellung des Kernmaterials und der primären Umhüllung (Modifizierung) der Oberfläche erfolgte wie in Beispiel 4.

### Aktivierung des magnetischen Nanomaterials:

Zur Herstellung einer wäßrigen 0,4 M NaIO₄-Lösung wurden 1,28 g (0,01 mol) Natriumperiodat in 15 ml Wasser gelöst.

Zur Einfügung zusätzlicher Carbonylgruppen wurden zu 2 g des magnetischen Nanomaterials, welches gemäß Beispiel 4 hergestellt wurde, 15 ml der 0,4 M NaIO₄-Lösung in sterilem Wasser hinzugegeben. Die Suspension wurde 2 Stunden lang bei Raumtemperatur unter milder Ultraschallbehandlung inkubiert. Das Produkt wurde sorgfältig mit sterilem Wasser unter Ultraschallbehandlung gewaschen und getrocknet.

### DOX-Chemisorption:

Zur Herstellung der wäßrigen DOX-Lösung wurden 18,44 mg DOX in 18 ml doppelt destilliertem und entionisiertem Wasser gelöst. Die resultierende Lösung wies einen pH-Wert von 6,475 auf.

2,5 % (w/v) O-(2-Aminoethyl)polyethylenglykol 3.000 in Krebspuffer und 1 % (w/v) Glycin in Wasser wurden wie in Beispiel 15 hergestellt.

12 ml der hergestellten DOX-Lösung wurden zu 0,25 g des aktivierten magnetischen Nanomaterials zugegeben. Die Suspension wurde bei Raumtemperatur unter Ultraschallbehandlung inkubiert. Zur Verfolgung der DOX-Chemisorption an der Oberfläche des aktivierten Nanomaterials wurden Proben des Überstandes entnommen. Nach einer 1 stündigen Inkubation wurden 2 ml der 2,5%igen (w/v) O-(2-Aminoethyl)polyethylenglykol 3.000-Lösung in Krebspuffer zugegeben, gefolgt von einer weiteren Inkubation unter milder Ultraschallbehandlung der Suspension für 1 Stunde. Anschließend wurden 2 ml der 1%igen Glycin-Lösung zugegeben, gefolgt von einer 1 stündigen Inkubation. Nach der gesamten, 3stündigen Inkubation wurde das Produkt magnetisch abgetrennt und sorgfältig mit Wasser gewaschen und gefriergetrocknet.

Die Konzentration an immobilisiertem DOX betrug in dem Produkt 10,5 mg DOX/g Träger (Carrier).

### Beispiel 17: Partikel mit immobilisiertem DOX

Die Herstellung des Kernmaterials und der primären Umhüllung (Modifizierung) der Oberfläche erfolgte wie in Beispiel 4.

### Aktivierung des magnetischen Nanomaterials:

Das gemäß Beispiel 4 hergestellte magnetische Nanomaterial wurde wie in Beispiel 7 aktiviert, um aktive Isocyanatgruppen einzufügen.

### DOX-Chemisoiption:

Die DOX-Chemisorption erfolgte wie in Beispiel 16. Nur zur Herstellung der wäßrigen DOX-Lösung wurde 18,4 mg DOX in 18 ml doppelt destilliertem und entionisierten Wasser gelöst. Die resultierende Lösung wies einen pH-Wert von 6,475 auf. Die Konzentration an immobilisiertem DOX betrug in dem Produkt 8,8 mg DOX/g Träger (Carrier).

### Beispiel 18: Partikel mit immobilisierten Paclitaxel

Die Herstellung des Kernmaterials und der primären Umhüllung (Modifizierung) der Oberfläche erfolgte wie in Beispiel 2.

### Aktivierung des magnetischen Nanomaterials:

Die Aktivierung des magnetischen Nanomaterials wurde wie in Beispiel 9 durchgeführt.

### Chemisorption von Paclitaxel:

Zur Herstellung einer Paclitaxeldichlormethanlösung wurden 10,5 mg Paclitaxel in 10 ml CH₂Cl₂ gelöst. Diese Stammlösung wurde 2 Minuten lang ultraschallbehandelt. Anschließend wurde 1 ml der Lösung entnommen und mit 9 ml trokkenem Dichlormethan verdünnt.

Zur Herstellung einer Polyethylenglykolisocyanat-Lösung (Molekulargewicht 3.400, von Sigma) wurden 50 mg in 1 ml trockenem Dichlormethan gelöst.

10 ml Paclitaxellösung wurden zu 0,2 g des aktivierten magnetischen Nanomaterials gegeben, gefolgt von einer Ultraschallbehandlung der Suspension. Anschließend wurden 20 ml TBT zugegeben und die Suspension für 2,5 Stunden inkubiert. Zur Neutralisierung von restlichen aktiven Gruppen wurde 1 ml Polyethylenglykolisocyanat-Lösung zugegeben, und die Suspension wurde 2 Stunden lang inkubiert.

Das Produkt wurde magnetisch abgetrennt, mit Dichlormethan sorgfältig gewaschen und getrocknet.

Die Konzentration an immobilisierten Paclitaxel betrug in dem Produkt 2,1 mg Paclitaxel/g Carrier.

Das Stabilisierungsverfahren wird zur Passivierung der Oberfläche durch Inhibierung aktiver Stellen aufgrund von Koordinierung oder Bindung der aktiven Modifizierungsgruppen durchgeführt. Als Folge werden Oxidierung und Hydratation der Oberflächenschicht minimiert bzw. verringert.

**Tabelle 1**

| Beispiel | Beispiel 1a | Beispiel 1b | Beispiel 2a | Beispiel 2b | Beispiel 3a | Beispiel 3b | Beispiel 3c | Beispiel 3d | Beispiel 4 | Beispiel 5 | Beispiel 6 |
|---|---|---|---|---|---|---|---|---|---|---|---|
| Kernzusammensetzung | 100 % Fe₃O₄ | 100 % Fe₃O₄ | 100% Fe₃O₄ | 98 % Fe,0, + 2 % 11-FeOOH | 99 % Te₃O₄ + 2% α-FeOOH | 99 % Fe₃O₄ + 1 % α-FeOOH | 100 % Fe,0, | 99 % Fe₃,O₄+ 1 % α-FeOOH | 100 % Fe₃O₄ | 100 % Fe₃O₄ | 98 % Fe,O, + 2 % α-FeOOH |
| Mittlere Kerngröße (nm) | 29 ± 16 | 24 | 39 ± 1:31 | 33 ± 33 | 24 ± 19 | 26 ± 22 | 28 | 24 ± 20 | 47 | 25 ± 18 | 29 ± 17 |
| Kerngröße (nm) Min. - Max. | 13-110 | 7-115 | 3-102 | 1-199 | 10-118 | 13 -114 | 8 -119 | 11-110 | 8-120 | 7-115 | 7-110 |
| Mittlere Kristallittengröße (nm) | 50.0 | 29,6 | 51.5 | 50.0 | 30.0 | 41.5 | 29,9 | 39.5 | 40 | 28,00 | 31.0 |
| Hülle | Vernetztes Dextran | Vernetztes Dextran | Vernetztes HES | Vernetztes HES | Kondensierte s γ-APS | Kondensierte s γ-APS | Kondensierte s γ-APS | Kondensiertes APS + Oxidiertes HES | Chitosan | Pfropf-copolymer AA + HEMA +EGDMA | Kondensiertes γ-APS |
| Süttigungsmagn Am²/g Fe | 115 ± 3 | 110,5 ± 4,1 | 114 ± 4 | 117 ± 5 | 134 ± 5 | 147 ± 4 | 114,5 ± 4,5 | 121 ± 3 | 117,3 ± 5,1 | 128 ± 4 | 122 ± 5 |
| Eisengehalt (Fe/g Troekengew.) | 0,70 | 0,26 | 0,693 | 0,65 | 0,598 | 0,693 | 0,44 | 0,631 | 0,4 | 0,650 | 0,608 |
| Gewichtsanteil der Hülle (g/g Trockengew.) | 0,03 | 0,65 | 0,04 | 0,10 | 0,17 | 0,04 | 0,39 | 0,13 | 0,45 | 0,10 | 0,16 |
| Hülle/Eisen-Verhältnis (gewichtsbez.) | 0,04 | 2,5 | 0,06 | 0,15 | 0,29 | 0,06 | 0,9 | 0,20 | 1,1 | 0,16 | 0,26 |

| | | | | | | | | | | | |
|---|---|---|---|---|---|---|---|---|---|---|---|
| HEMA= 2-Hydroxyethylmethacrylat oder Glykolmetacrylat AA = Acrylamid EGDMA= EthylenglyKoldimethacrylat HES= Hydroxyethytstärke γ-APS = γ-Aminopropyltriethoxysilan | | | | | | | | | | | |

**Tabelle 2**

| **Beispiel** | | **Beispiel 7** | **Beispiel 8** | **Beispiel 9** | **Beispiel 10** | **Beispiel 11** | **Beispiel 12** | **Beispiel 13** | **Beispiel 14** | **Beispiel 15** | **Beispiel 16** | **Beispiel 17** | **Beispiel 18** |
|---|---|---|---|---|---|---|---|---|---|---|---|---|---|
| **Partikel gem. Beispiel** | | 1a | 2 | 2 | 3a | 3a | 3a | 3a | 3a | 4 | 4 | 4 | 2 |
| **Aktivierungsprozeß** / **Spacer** | | HMDIC | HMDIC | HMDIC | HMDIC | HMDIC | HMDIC | HMDIC | HMDIC | Periodat oxidation von HES | Periodat oxidation vonHES | HMDIC | HMDIC |
| **Aktive Gruppen für Immobilisierung** | | -*NCO* | *-NCO* | *-NCO* | *-NCO* | *-NCO* | *-NCO* | -*NCO* | *-NCO* | >*CO* | >*CO* | *-NCO* | *-NCO* |
| **Neutralisierung der restlichen aktiven Gruppen** | | EG | EG | EG | APEG | APEG | APEG | APEG | APEG | APEG+Gl | APEG+Gl | APEG+Gl | APEG |
| **Eisengehalt, g Fe/g Trockengewicht** | | 0,67 | 0,68 | 0,65 | 0,57 | 0,57 | 0,52 | 0,66 | 0,66 | 0,68 | 0,66 | 0,60 | 0,46 |
| **Immobilisiertes BAM** | | Doxorubicin | | | | | | | | | | | Paclitaxel |
| **Konzentration immobilisiertes BAM, mg/g Träger** | | **2,5** | 4,8 | 27 | 25,1 | 6,1 | 1,2 | 0,4 | 0,16 | 1,8 | 10,5 | 8,8 | 2,1 |
| **Konzentration immobilisiertes BAM (Gewicht BAM/Gewicht Hülle, %)** | | 3,3% | 7,9% | 25,3% | 11,9% | 2,9% | 0,4% | 0,5% | 0,2% | 2,9% | 11,9% | 5,3% | 0,6% |
| **Cytotoxizität gegen KB-Zellen** | **IC₅₀, mkg /ml** | 0,023± 0,002 | 0,039± 0,006 | 0,020± 0,001 | 0,007± 0,001 | 0,009± 0,001 | 0,017± 0,001 | 0,012± 0,001 | 0,044± 0,002 | 0,009± 0,001 | 0,069± 0,003 | 0,0084± 0,0002 | 0,0029± 0,0017 |
| | **IC50_{frei}/ IC50_{konjugiert}** | 0,56 | 0,33 | 0,8 | 0,93 | 0,72 | 0,38 | 0,54 | 0,15 | 0,14 | 0,02 | 0,27 | 0,24 |
| **Cytotoxizität gegen A2780-Zellen** | **mkg /ml** | 0,010± 0,001 | 0,021± 0,001 | 0,237± 0,015 | 0,016± 0,001 | 0,002± 0,0005 | 0,013± 0,004 | 0,004± 0,001 | 0,003± 0,0008 | 0,027± 0,010 | 0,047± 0,004 | 0,0042± 0,0001 | 0,0096± 0,0005 |
| | **IC50_{frei}/ IC50_{konjugiert}** | 0,97 | 0,44 | 0,01 | 0,56 | 1,48 | 0,27 | 0,82 | 1,03 | 0,03 | 0,02 | 0,59 | 0,017 |
| **Affinität in bezug auf** | | nt | nt | nt | + | + | + | + | + | nt | nt | nt | nt |

| | | | | | | | | | | | | | |
|---|---|---|---|---|---|---|---|---|---|---|---|---|---|
| HMDIC=1,6-Hexamethylendüsocyanat BAM=bioaktives Moiekül EG=EthlenglyKol APEG=Aminopolyethylenglykol 3'000; Gl=Glycine Nt=nicht geprüft / nicht getestet | | | | | | | | | | | | | |

**Tabelle 3**

| Beispiel | Gewicht der aktivierten 3a-Probe, g | DOX-Lösung | V_{DOX-Lösung} ml | T_{inc}, min | [DOX]ᵢₘₘ, mg DOX/g Partikel |
|---|---|---|---|---|---|
| Referenz | 0,257 | - | - | - | 0 |
| Beispiel 10 | 0,252 | DOX-Beispiel 10 | 11 | 180 | 25,1 |
| Beispiel 11 | 0,253 | DOX-Beispiel 11 | 11 | 180 | 6,1 |
| Beispiel 12 | 0,251 | DOX-Beispie112 | 10 | 180 | 1,2 |
| Beispiel 13 | 0,257 | DOX-Beispie113 | 10 | 120 | 0,4 |
| Beispiel 14 | 0,258 | DOX-Beispiel 14 | 10 | 120 | 0,16 |

## Patentansprüche

1. Partikel zur therapeutischen Anwendung in einem Arzneimittel, insbesondere zur Bindung mit einem therapeutischen Wirkstoff, jeweils mit einem zu mindestens 95 Gew.-% aus Eisenoxid bestehenden, nanokristallinen magnetischen Kern und einer den Kern umschließenden Hülle,
wobei die Hülle passivierend und/oder für Sauerstoff undurchlässig ausgebildet ist und an die Hülle mindestens ein therapeutischer Wirkstoff kovalent gebunden ist und
wobei die spezifische Sättigungsmagnetisierung der Partikel mindestens 110 Am²/kg Eisen beträgt.

2. Partikel nach Anspruch 1, **dadurch gekennzeichnet, daß** die spezifische Sättigungsmagnetisierung der Partikel mindestens 114 Am²/kg Eisen, vorzugsweise mindestens 117 Am²/kg Eisen, insbesondere mindestens 120 Am²/kg Eisen, beträgt und/oder daß der Kern Eisen als Ion enthält und/oder daß der Kern zu mindestens mindestens 99 Gew.-%, aus Eisenoxid besteht, insbesondere wobei das Eisenoxid zumindest teilweise aus magnetischem Eisenoxid besteht, insbesondere ausgewählt aus der Gruppe von Magnetit, Ferrit, Hämatit, Maghemit, Geothit, Lepidocrocit und/oder Ferroxyhyt, und/oder daß der Kern Magnetit enthält oder daraus besteht und/oder daß der Kern einen mittleren Durchmesser von 3 bis 200 nm, vorzugsweise von 10 bis 100 nm, insbesondere von 20 bis 52 nm, aufweist und/oder daß der Kern ferro-, ferri- oder superparamagnetisch ist und/oder daß der Kern monokristallin oder polykristallin ist.

3. Partikel nach einem der voranstehenden Ansprüche, **dadurch gekennzeichnet, daß** die Partikel 26 bis 70 Gew.-% Eisen, bezogen auf das Trockengewicht der Partikel, enthalten und/oder daß die Partikel eine spezifische Sättigungsmagnetisierung von mindestens 85 %, insbesondere mindestens 90 %, der spezifischen. Sättigungsmagnetisierung des nicht superparamagnetischen Bulk-Kernmaterials aufweisen.

4. Partikel nach einem der voranstehenden Ansprüche, **dadurch gekennzeichnet, daß** die Hülle gasdicht ausgebildet ist und/oder daß die Hülle durch Adsorption und Vernetzung von Monomeren gebildet ist und/oder daß die Hülle ein Polymer enthält oder daraus besteht und/oder daß die Hülle durch Kondensation von Silanen gebildet ist und/oder daß die Hülle vernetztes Dextran, Hydroxyethylstärke, kondensiertes Silan, Chitosan, ein Copolymer von Acrylamid und Methacrylat oder Gemische von zwei oder mehreren dieser Verbindungen enthält oder hieraus besteht.

5. Partikel nach einem der voranstehenden Ansprüche, **dadurch gekennzeichnet, daß** der Wirkstoff ein Antikrebsmittel und/oder ein Antibiotikum ist, insbesondere wobei der Wirkstoff ausgewählt ist aus der Gruppe von Doxorubicin, Taxol und Steroidhormonen, und/oder daß der Wirkstoff zelloberflächenaktiv ist und/ oder daß der Wirkstoff intrazellular oder intranuklear wirkend ist und/oder daß der Wirkstoff ein Molekulargewicht von 20 bis 200.000 g/mol, insbesondere 500 bis 600 g/mol, oder eine Kettenlänge von 10 bis 600.000 Atomen, insbesondere 500 bis 600 Atomen, aufweist und/oder daß der Wirkstoff und die Partikel über funktionelle Gruppen, insbesondere -OH, -COOH, =C=O, -NH₂, -S-OH oder -SH, gebunden sind.

6. Arzneimittel oder Diagnostikum, umfassend Partikel nach einem oder mehreren der voranstehenden Ansprüche.

7. Verwendung von Partikeln nach einem der Ansprüche 1 bis 5 zur Herstellung eines Arzneimittels zur Therapie von Tumor-, insbesondere Krebserkrankungen, von bakteriellen Erkrankungen, von rheumatischen Erkrankungen, von fokalen Anfallsleiden, von neurologischen Erkrankungen, von Thrombosen, von gynäkologischen Erkrankungen, von Erkrankungen des männlichen Sexualorgans, von Erkrankungen des Ohres oder von fokalen Knochenerkrankungen.

## Claims

1. Particles for therapeutic use in a drug, in particular for bonding with a therapeutic active ingredient, in each case having a nanocrystalline magnetic core consisting at least 95 % by weight of iron oxide and a casing surrounding the core,
wherein the casing is formed passively and/or impermeable to oxygen, at least substantially, and at least one therapeutic active ingredient is covalently bonded to the casing, and
wherein the specific saturation magnetisation of the particles is at least 110 Am²/kg iron.

2. The particles according to claim 1, **characterised in that** the specific saturation magnetisation of the particles is at least 114 Am²/kg iron, preferably at least 117 Am²/kg iron, in particular at least 120 Am²/kg iron, and/or **in that** the core contains iron as an ion, and/or **in that** the core consists at least 99 % by weight of iron oxide, in particular the iron oxide consisting at least in part of magnetic iron oxide, in particular selected from the group of magnetite, ferrite, hematite, maghemite, goethite, lepidocrocite and/or feroxyhyte, and/or **in that** the core contains magnetite or consists thereof, and/or **in that** the core has a mean diameter of 3 to 200 nm, preferably of 10 to 100 nm, in particular of 20 to 52 nm, and/or **in that** the core is ferromagnetic, ferrimagnetic or superparamagnetic, and/or **in that** the core is monocrystalline or polycrystalline.

3. The particles according to any one of the preceding claims, **characterised in that** the particles contain 26 to 70 % by weight of iron based on the dry weight of the particles, and/or **in that** the particles have a specific saturation magnetisation of at least 85 %, in particular at least 90 % of the specific saturation magnetisation of the bulk core material, which is not superpara-magnetic.

4. The particles according to any one of the preceding claims, **characterised in that** the casing is gastight, and/or **in that** the casing is formed by adsorption and crosslinking of monomers, and/or **in that** the casing contains a polymer or consists thereof, and/or **in that** the casing is formed by condensation of silanes, and/or **in that** the casing contains crosslinked dextran, hydroxyethyl starch, condensed silane, chitosan, a copolymer of acrylamide and methacrylate or mixtures of two or more of these compounds or consists thereof.

5. The particles according to any one of the preceding claims, **characterised in that** the active ingredient is an anti-cancer drug or an antibiotic, in particular the active ingredient being selected from the group of doxorubicin, taxol and steroid hormones, and/or **in that** the active ingredient is cell surface-active, and/or **in that** the active ingredient acts in an intracellular or intranuclear manner, and/or **in that** the active ingredient has a molecular weight of 20 to 200,000 g/mol, in particular 500 to 600 g/mol, or a chain length of 10 to 600,000 atoms, in particular 500 to 600 atoms, and/or **in that** the active ingredient and the particles are bonded by functional groups, in particular - OH, -COOH, =C=O, -NH₂, -S-OH or -SH.

6. A drug or diagnostic agent, containing particles according to one or more of the preceding claims.

7. A use of particles according to any one of claims 1 to 5 to produce a drug for the treatment of tumours, in particular cancers, bacterial diseases, rheumatic disorders, focal seizure disorders, neurological disorders, thrombosis, gynaecological disorders, disorders of the male sex organ, ear disorders or focal bone disorders.

## Revendications

1. Particules destinées à une application thérapeutique dans un médicament, notamment à former une liaison avec un principe actif thérapeutique; chacune d'entre elles ayant un noyau magnétique nanocristallin constitué à au moins 95 % en poids d'oxyde de fer et une enveloppe entourant ledit noyau,
ladite enveloppe étant réalisée de façon à exercer un effet de passivation ou à être imperméable à l'oxygène, et au moins un principe actif thérapeutique étant lié de façon covalente à ladite enveloppe, et
la valeur de l'aimantation à saturation spécifique aux particules étant d'au moins 110 Am²/kg de fer.

2. Particules selon la revendication 1, **caractérisées en ce que** la valeur de l'aimantation à saturation spécifique aux particules est d'au moins 114 Am²/kg de fer, préférentiellement d'au moins 117 Am²/kg de fer, notamment d'au moins 120 Am²/kg de fer, et/ou que ledit noyau contient du fer sous forme ionique, et/ou que ledit noyau est constitué à au moins 99 % en poids d'oxyde de fer, ledit oxyde de fer étant au moins partiellement constitué d'oxydes de fer magnétiques, choisis notamment dans le groupe de la magnétite, la ferrite, l'hématite, la maghémite, la géothite, la lépidocrocite et/ou la feroxyhyte, et/ou que ledit noyau contient de la magnétite ou en est constitué, et/ou que ledit noyau présente un diamètre moyen de 3 à 200 nm, préférentiellement de 10 à 100 nm, notamment de 20 à 52 nm, et/ou que ledit noyau est ferro-, ferri- ou superparamagnétique, et/ou que ledit noyau est monocristallin ou polycristallin.

3. Particules selon l'une des revendications précédentes, **caractérisées en ce que** lesdites particules contiennent 26 à 70 % en poids de fer, par rapport au poids sec desdites particules, et/ou que lesdites particules présentent une valeur spécifique d'aimantation à saturation d'au moins 85 %, notamment d'au moins 90 %, de la valeur d'aimantation à saturation spécifique au matériau non-superparamagnétique remplissant le volume du noyau.

4. Particules selon l'une des revendications précédentes, **caractérisées en ce que** ladite enveloppe est réalisée de façon à être étanche aux gaz, et/ou que ladite enveloppe est réalisée par adsorption et réticulation de monomères, et/ou que ladite enveloppe contient un polymère ou en est constitué, et/ou que ladite enveloppe est réalisée par condensation de silanes, et/ou que ladite enveloppe contient du dextrane réticulé, de l'hydroxyéthylamidon, du silane ayant subi une condensation, du chitosane, un copolymère d'acrylamide et de méthacrylate ou des mélanges de deux ou plusieurs desdites composés ou qu'elle en est constituée.

5. Particules selon l'une des revendications précédentes, **caractérisées en ce que** ledit principe actif est un agent anticancéreux ou un agent antibiotique, ledit principe actif étant notamment choisi dans le groupe de la doxorubicine, du taxol et des hormones stéroïdiennes, et/ou que ledit principe actif agit sur la surface cellulaire, et/ou que ledit principe actif agit de façon intracellulaire ou intranucléaire, et/ou que ledit principe actif présente une masse moléculaire de 20 à 200 000 g/mol, notamment de 500 à 600 g/mol, ou une longueur de chaîne de 10 à 600 000 atomes, notamment de 500 à 600 atomes, et/ou que ledit principe actif et lesdites particules sont liés au travers de groupes fonctionnels, s'agissant notamment de -OH, -COOH, =C=O, -NH₂, -S-OH ou -SH.

6. Médicament ou agent diagnostic, comprenant des particules selon l'une ou plusieurs des revendications précédentes.

7. Utilisation de particules selon l'une des revendications 1 à 5 pour préparer un médicament destiné à la thérapie d'affections relatives à des tumeurs, s'agissant notamment d'affections cancéreuses, d'affections bactériennes, d'affections rhumatismales, de crises partielles d'épilepsie, d'affections neurologiques, de thromboses, d'affections gynécologiques, d'affections de l'organe sexuel masculin, d'affections de l'oreille et d'affections osseuses non-généralisées.
